# EUROPEAN PATENT APPLICATION

(11) **EP 1 645 283 A1**
(43) Date of publication of application: **12.04.2006**
(21) Application number: 04024103.6
(22) Date of filing: 08.10.2004
(51) Int. Cl.: A61K 39/00, A61K 39/295, A61K 39/05

(54) **Combination vaccine**

(71) Applicant: Chiron Behring GmbH & Co. KG, 35006 Marburg (DE)
(72) Inventor: Bröker, Michael, Dr., 35041 Marburg (DE)
(74) Representative: UEXKÜLL & STOLBERG

(57) **Abstract**

The present invention relates to the combination of antigens directed against bacteria and viruses, their uses and the preparation of medicaments in order to confer protection against infectious diseases. In particular, the invention relates to a combination vaccine comprising at least one antigen of Clostridium tetani, at least one antigen from Corynebacterium diphtheriae, and at least one antigen from the TBE-flavivirus suitable to confer seroprotection against diseases and medical conditions caused by these pathogenic organisms.

## Description

The present invention relates to the combination of antigens directed against bacteria and viruses, their uses and the preparation of medicaments containing these combination of antigens to confer protection against infectious diseases. In particular, the invention relates to a combination vaccine comprising at least one antigen conferring protection against tetanus, at least one antigen conferring protection against diphtheria and at least one antigen conferring protection against tick-borne encephalitis (TBE). According to a preferred embodiment of the invention, a combination is provided comprising at least one antigen of Clostridium tetani, at least one antigen from Corynebacterium diphtheriae, and at least one antigen from the tick-borne encephalitis virus.

The induction of specific immunity to infectious diseases was one of the most important milestones in modern medicine. Today, a vast number of different vaccines are known in the art for the prophylactic protection of humans and animals. Among the vaccines, which are in wide use are those against tetanus and diphtheria. Tetanus and diphtheria vaccines usually contain inactivated toxins (toxoids) as antigens, which are capable of inducing protection upon administration. These toxoids are generally adsorbed on aluminium salts e.g. aluminium hydroxide, to enhance their immunogenicity.

For the matter of convenience, the tetanus and diphtheria antigens can be combined to a tetanus/diphtheria-combination vaccine which induces a protective immunity against both tetanus and diphtheria. In this way, multiple injections as well as a large total injection volume can be avoided. Several combination vaccines are already known in the art, for example, vaccines against diphtheria (D), tetanus (T) and pertussis (T; combination referred to DTP), vaccines against measles and mumps (MM) or against measles (M), mumps (M) and rubella (R; combination referred to as MMR). Further, also pentavalent vaccines such as DTP together with Hepatitis B virus (HBV) antigen and Haemophilus influenzae type B antigen (Hib) are in practical use. Further, multivalent vaccines containing antigens from acellular pertussis (aP) and inactivated polio virus (IPV) have been prepared. For example the use of the hexavalent vaccine DTaP-HBV-IPV-Hib containing antigens for conferring protection against diphtheria, tetanus, pertussis, Hepatitis B, Polio and Haemophilus influenzae type B infections was described in the prior art (Scandinavian Journal of Infectious Diseases, 2004, 36 (8), pp. 585-592).

A prerequisite for combination vaccines is a lack of "competing" antigens and a high compatibility with respect to the subject to be immunized. According to international standard applied in connection with immunization, a combined vaccine should confer a protection which is comparable to that achieved by separate vaccinations. However, the combination of antigens is a complicated process which is associated with a number of problems and uncertainties. As the individual components of a combination vaccine are not inert substances, the combination of various components into one mixture can negatively influence the immunogenicity of the individual antigenic components. For example, interactions between the different antigens or between the antigens and other components typically used in such vaccines can occur due to differences in charge, chemical residues, detergents, formaldehyde, concentration of ions etc. These changes can occur instantaneously after contacting the different antigens with each other or with other substances usually present in vaccine formulations. Moreover, these changes can also occur with a significant delay after mixing, for example during storage, shipping, etc. However, it cannot be predicted to which extent the immunogenicity of individual antigens may be influenced by mixing them to one combination. As a consequence, a situation can occur in which one or more antigens of the vaccine only confer an insufficient seroprotection against one or more of diseases which renders the product unsuitable for medical practise.

For example, Zott (Bundesgesundhbl. issue 12/1997, 498-501) reports, that replacing a whole cell pertussis component by an acellular pertussis antigen (DaPT) leads to a decrease in potency from 370 IU/dose to 84 IU/dose of the tetanus component in a combination vaccine consisting of whole cell pertussis, diphtheria toxoid and tetanus toxoid (DPT).

Similarly, a combination vaccine consisting of Haemophilus influenzae type b conjugate antigen, diphtheria and tetanus toxoid, acellular pertussis antigens and inactivated poliovirus antigens (DTaP-Hib-IPV) was shown in clinical trials to exhibit a reduced antibody formation against the Hib antigen when compared to a combination of DTaP combination given simultaneously with monovalent IPV and Hib vaccines at different sites of the body (Eskola et al., Lancet 1996; 348: 1688-1692). The consequences of such interactions leading to a loss in seroprotection can be dramatic. McVernon et al. (BMJ, 2004, 329, pp. 655-658) even blame the broad use of such a less immunogenic combination vaccines containing a Haemophilus influenzae antigen together with an a cellular pertussis component in the years 2000-2001 in the UK to be one factor of the overall increase in invasive infections with Haemophilus influenzae type B in the UK since 1998.

These results clearly demonstrate that the production of effective combination vaccines is a complex matter which depends on multidimensional interactions between the individual components of the vaccine and does not allow to extrapolate any effect of the components observed when administered separately. Thus, there is a need for combination vaccines which confer protection against several infectious diseases. This need is fulfilled by a combination vaccine according to the invention.

### Brief Summary of the invention

It has now surprisingly been found by the applicants that a combination vaccine comprising at least one antigen conferring protection against tetanus, at least one antigen conferring protection against diphtheria, and at least one antigen conferring protection against tick-borne encephalitis is suitable to confer an effective seroprotection against the symptoms normally associated with tetanus, diphtheria and tick-borne encephalitis-virus infections. Particularly, a combination vaccine comprising at least one antigen of Clostridium tetani, at least one antigen from Corynebacterium diphtheriae, and at least one antigen from the TBE-virus is provided. The vaccine according to the invention shows an excellent immunological protection. Moreover, the combination vaccine according to the present invention can be combined with certain other antigens without significantly reducing effectiveness of the vaccine composition.

### Detailed Description of the Invention

Diphtheria is an infectious disease which is caused by Corynebacterium diphtheriae. Certain strains of this organism produce a heat-labile polypeptide toxin having a molecular weight of approximately 62000 Da. The toxin secreted into the medium belongs to the so-called "A-B-toxins" which consist of two fragments. Fragment A (approximately 22000 Da) represents the lethal toxin which is capable to inactivate the elongation factor 2 in the target cell in the presence of NAD. Thereby, the toxin leads to an inhibition of protein synthesis and to cell death. On the other hand, fragment B (about 40000 Da) is involved in binding to the target cell and transporting fragment A through the cytoplasmic membrane into the cytoplasm, where the toxin exhibits its effect. Notably, all strains of Corynebacterium diphtheriae which produce the exotoxin comprise a specific bacteriophage (designated as beta phage) which encodes the toxin.

Generally, vaccine booster doses approximately every 10 years are indicated for the maintenance of the acquired immunity. According to the recommendations set forth by the WHO, diphtheria vaccine is usually administered as a triple vaccine (DTP) for preschool children for primary and reinforcing ("booster") immunization while the bivalent vaccine (DT) is employed for booster doses in these preschool children. The diphtheria toxoid amount used in such vaccines is about 20 Lf/ml (Lf is the abbreviation of Limes flocculationis that is defined in detail below) or more and has a potency of at least 20 to 50 International Units/dose (IU/dose). This amount is designated as the paediatric form of the toxoid (DT).

Because such a high concentration of diphtheria antigen may induce a relative high reactogenicity in persons older than five years, diphtheria vaccines for older persons usually contain a smaller antigen amount of diphtheria toxoid. From five to seven years of age onwards, a concentration of the toxoid (Td) suitable for adults form is used (adult administration form or adult form), containing much less toxoid than the paediatric form. For example, the monovalent diphtheria vaccine for adults produced by Chiron Behring GmbH & Co KG contains 1.5 Lf diphtheria toxoid and exhibits at least 2 IU/dose.

This vaccine can be used to vaccinate persons aged five years or older and who have had a complete primary vaccination against diphtheria during their infancy and who want to boost their immune response. In many countries, a booster vaccination against diphtheria is recommended every 10 years. Because the antibody titre against tetanus also wanes over time, a booster vaccination against tetanus is also recommended every 10 years. If the recommendation to boost the tetanus and diphtheria immune response is not complied, these persons are at risk to develop diphtheria or tetanus upon infection by Corynebacterium diphtheriae or Clostridium tetani.

Tetanus is caused by the pathogenic microorganism Clostridium tetani. Microorganisms of the genus Clostridium are only capable to grow under anaerobic conditions. Clostridium tetani synthesizes several toxins, the most important of which is tetanospasmin, a heat-labile protein with a molecular weight of 150000 Da. Tetanospasmin is an extraordinary strong bacterial toxin; an amount of 10⁻⁴ µg of pure toxin is sufficient to kill a mouse within 48 hours. As the diphtheria toxin, tetanus toxin belongs to the so-called "A-B-toxins" and consists of two sub-units, a heavy chain of about 100000 Da and a light chain of about 50000 Da. The heavy chain is responsible for binding to the target cells, whereas the light chain represents the toxic component.

The clinical symptoms of tetanus are caused by the synthesis of tetanus toxin in the infected organism. Clostridium tetani can grow in deep wound punctures that become anaerobic. Although the microorganism itself does not invade the body from the initial site of infection, the toxin produced can spread and cause severe neurological disturbance which can lead to death. Upon entry of the toxin into the central nervous system, it becomes fixed to nerve synapses by binding to one of the lipids in the membrane. Specifically, the toxin blocks the activity of the nerve factor that allows relaxation of the muscles, resulting in constant firing of motor neurons which leads to spastic paralysis typically associated with tetanus.

Tick-borne encephalitis (TBE) virus is a species of the tick-borne group within the flaviviruses. The TBE-virus (also designated Central European Encephalitis virus or Russian spring-summer encephalitis virus or in German: Frühsommer-Meningo-Enzephalitis-Virus (FSME-virus)) causes a variety of clinical symptoms in humans including subclinical infections, mild or severe fever and meningitis, meningoencephalitis and meningo-encephalo-myelitis with serious sequelae (Kaiser, Brain 1999; 122: 2067-2078). Many regions in Europe and Asia are endemic for TBE and therefore, TBE vaccination is recommended in many European and Asian countries (Dumpis et al., Clin. Infect. Dis. 1999; 28: 882-890; Süss, Vaccine 2003; S1: 19-35).

The TBE vaccines regularly contain inactivated virus, mostly the envelope glycoprotein E, and the viral antigen - as with the T and D/d antigens - is also adsorbed to aluminium salts. The TBE vaccines available, consist of inactivated and purified viral antigens from either strain Neudörfl (Heinz et al., J. Med. Virol. 1980; 6: 213-221) or strain K23 (Klockmann et al., J. Biol. Standard. 1989; 17: 331-342). Based on serological and genetic analyses of isolates from Europe, Siberia and Far East Asia, TBE-virus has been subdivided into three closely related subtypes, but the degree of variation in the amino acid level of the glycoprotein E antigen, which confer immune protection, is low (Ecker et al., J. Gen. Virol. 1999; 80: 179-185) and therefore the TBE vaccines either based on the strain Neudörfl or K23 are interchangeable and both induce protection against a broad variety of TBE-virus strains.

The originally developed TBE vaccines may induce systemic side effects, e.g. fever, in young children and therefore TBE vaccines with a reduced amount of TBE antigen have been developed for the immunization of children (Girgsdies and Rosenkranz, Vaccine 1996; 14: 1421-1428; Zent et al., Vaccine 2003; 21: 3584-3952). For example, the TBE vaccine Encepur® for adults (Chiron Behring GmbH & Co KG) contains 1.5 µg antigen per dose, while Encepur® for children (Chiron Behring GmbH & Co KG) consists of 0.75 µg per dose and is applied to children from 1 to 11 years of age. Notably, in the case of TBE, no international calibration exist which would allow for reciting to the antigen amount in terms of its potency. Consequently, the amount of antigen used in the case of TBE is adressed in µg. Interestingly, the reactogenicity profile in young and adults persons of the TBE vaccines and the above mentioned diphtheria or tetanus/diphtheria vaccines behave quite different. While the side effects of a vaccine containing a high diphtheria antigen amount are more common in adults compared to children, the number of side reactions for the TBE vaccines is increased in children compared to adults, when the antigen amount is high.

The primary immunization against TBE consists of three vaccine doses, preferentially given at day 0, month 1-3 (after the first dose) and month 9-12 (after the second dose) for both the vaccines either based on strain Neudörfl or K23 and according to the Specific Product Characteristics of the manufacturers. Booster vaccinations are recommended every three years for persons who are at risk of TBE infection. Based on these recommendations, persons living in regions, in which the TBE-virus is endemic, may get 25 or more TBE vaccinations during their life in order to ensure protection against TBE.

Recently, a serological survey revealed, that protection against TBE upon immunisation might last for longer than three years (Rendi-Wagner et al., Vaccine 2004; 22, pp. 2743-2749) and therefore, the Vaccination Committee (Impfausschuss) of the Austrian vaccination advisory board "Oberster Sanitätsrat" in Austria recommends from 2004 onwards to expand the booster intervals for persons up to 59 years of age from three to five years upon the first booster vaccination has been carried out (Hörandl, Impfplan 2004, Welche Neuerungen gibt es? Jatros Vaccines, 1/2004, 4-5; Hörandl, FSME-Impfung. Impfschutz auf fünf Jahre verlangert. Jatros Vaccines 1/2004, 6-7). One may expect that other countries will follow these recommendations. It cannot even be excluded, that on the long term, the booster intervals for TBE vaccination may be extended to even ten years. In this case, a combination vaccine conferring protection against tetanus, diphtheria and TBE is of particular advantage, since the booster interval for the three diseases would be identical.

### Advantages of the combination vaccine of the invention

The primary immunisation against tetanus and diphtheria is generally carried out in infants aged 3 to 12 months and booster vaccinations are recommend at school entry and about primary school leave and then every 10 years. While humans become older, the efficacy of the immune system decreases and therefore, in Austria, booster immunisation against tetanus and diphtheria for persons over 60 years are recommended every 5 years in order to guarantee a sufficient immune response and a level of protective antibody titre against tetanus and diphtheria, while in Germany, the vaccination advisory board, the STIKO (Ständige Impfkommission am Robert Koch-Institut) recommends 10 years- booster intervals also for the elderly. Primary vaccination against TBE is recommended for children aged 3 years and older, adolescents and adults living in or going to TBE-virus endemic regions and booster vaccinations are recommended every three years and without any differences regarding the vaccination intervals for various age groups.

Whenever a booster vaccination against tetanus, diphtheria and TBE should be done in persons aged five years or older, two vaccine shots have to be carried out actually, namely a Td and a TBE vaccination. These two vaccines can be individually given either two to four weeks apart and concomitantly at two sites of the body, e.g. the left and the right upper arms. According to the present invention, the protection against tetanus, diphtheria and TBE can be facilitated by a combination vaccine, consisting of T, d and TBE antigens. As used herein, this combination vaccine is termed Td-TBE. Persons aged 5 to 11 years will preferably receive a Td-TBE combination vaccine with a reduced TBE antigen amount as it is already applied by the current monovalent TBE vaccines (Zent et al., Vaccine 2003; 21: 3584-3952; Ehrlich et al., Int. J. Med. Microbiol. 2004; 37: 126-127). For most of the other potential vaccinees, who are aged 12 years or older, the Td-TBE vaccine should consist of the TBE antigen amount, which is already used for immunisation of older children, adolescents and adults (Zent et al., Vaccine, 2003; 21: 738-741; Ehrlich et al., Vaccine 2003; 22: 217-223). In the context of the present invention, the adult form of the diphtheria toxoid vaccine with the reduced antigen concentration is generally designated as "d", while the paediatric form having the high amount of diphtheria toxoid used in vaccines for children is termed "D". For example, the DT-Impfstoff Behring für Kinder (a DT adsorbate vaccine for children; Chiron Behring GmbH & Co KG) contains 20 Lf tetanus toxoid with a potency of at least 40 IU/dose and 20 Lf diphtheria toxoid with a potency of at least 30 IU/dose. In comparison, the combination vaccine Td-pur® (Chiron Behring GmbH & Co KG) contains 20 Lf tetanus toxoid with a potency of at least 20 IU/dose and only 1.5 Lf diphtheria toxoid having a potency of at least 2 IU/dose.

This new combination vaccine fulfils the wish of patients, because the number of injections which are necessary to maintain a protective immune status regarding tetanus, diphtheria and TBE can be reduced significantly. In addition, this new combination vaccine supports efforts to bring the TBE vaccine away from being only a seasonal vaccination and given preferred during the spring-time in order to be protected against TBE during the following summer.

When a Td booster vaccination should be given to a person in autumn, because the interval of 10 years or more after the last Td vaccination is exceeded, subjects often refrain to get simultaneously vaccinated also against TBE due to fear of two needle injections at the same time, even when a TBE booster vaccination is indicated. In such a situation, many persons prefer to become vaccinated during springtime of the following year, but unfortunately these immunizations are then very often forgotten and the persons remain unprotected. One should have in mind, that infections by the TBE-virus can occur early in the year, e.g. the first reported TBE-virus infection in Austria in the year 2001 was as early as in February (Waldner et al., Wien. Klin. Wochenschr. 113, 454-458 (2001)). This example shows, that the delay of a TBE booster vaccination can lead to an infection, which could have been prevented by a precocious vaccination and a Td-TBE combination vaccine increases the acceptance of TBE booster vaccinations.

Whenever a Td vaccination is indicated, the use of the new Td-TBE vaccine is advisable for persons of risk for TBE infections and if the last vaccination against TBE was at least three years ago.

The value of the new Td-TBE combination vaccine can significantly reduce the number of immunisations for tetanus, diphtheria and TBE for persons living in TBE endemic areas. Assuming, that a person aged 20 years will have up to his/her 80th year of age about 7 Td booster vaccinations according to the actual German vaccination recommendations and 13 TBE booster injections according to a foreseen five-year booster interval for TBE vaccination, the combined Td-TBE booster vaccination would reducethe number of vaccinations by a total of seven injections. That means, the number of injections can be reduced by 35 %. Practically, the 20 year old person would be immunised with the Td-TBE combination vaccine by the age of 20 years to booster against tetanus, diphtheria and TBE. The next TBE booster would be given when aged 25 with a monovalent TBE vaccine. Aged 30, the person would receive again a Td-TBE booster injection, followed by a monovalent TBE vaccination when aged 30 years, etc.

In the case, that the recommendations for TBE booster vaccination will change sometime from three or five years to ten years, the management of booster immunisation against tetanus, diphtheria and TBE will be even more easier and practicable with the new Td-TBE combination vaccine and will reduce the injections by 50 %. Reduction of the number of injections not only increases the acceptance of vaccinations, but also reduces the number of side effects and therefore are an additional medicinal progress.

Even if the person to be vaccinated has not obtained a TBE vaccination so far, one can combine the Td booster immunization with the first dose of a primary TBE immunization using this new Td-TBE vaccine. The next two TBE immunizations to complete the primary TBE vaccination are then given by the monovalent TBE vaccine formulation.

The Td-TBE combination vaccine according to the invention not only supports the acceptance for TBE vaccinations, but is also a measure to enhance the general booster vaccination rates to protect against tetanus and diphtheria. There are big immunisation gaps for tetanus and diphtheria especially in the group of adults and elderly people (Hammer et al., InfFo 1/97, 35-37), such that many people have no or only a limited protection against tetanus and diphtheria. Although most of the people are generally willing and interested to become vaccinated, the reason for the low coverage rate may be seen in the fact, that patients and physicians both do not mostly think about a tetanus and/or diphtheria vaccination on the occasion of a patient's visit. If a patient has the concrete wish to become immunised against TBE, e.g. because he/she wants to travel from a TBE non-endemic to an endemic region, this is a good opportunity to vaccinate with the Td-TBE combination vaccine if indicated instead of only with the monovalent TBE vaccine.

It is generally agreed that there are no maximal intervals between vaccinations. If the recommended interval for booster vaccination against tetanus, diphtheria and/or TBE is exceeded, one single injection with this new Td-TBE vaccine is sufficient to ensure protection against these three infections.

According to the present invention, a combination vaccine is provided which comprises antigens conferring protection against at least three different infectious diseases. Specifically, the invention relates to a combination vaccine comprising at least one antigen conferring protection against tetanus, at least one antigen conferring protection against diphtheria and at least one antigen conferring protection against tick-borne encephalitis. According to a preferred embodiment of the invention, a combination vaccine is provided comprising at least one antigen from Clostridium tetani, at least one antigen from Corynebacterium diphtheriae and at least one antigen from the TBE-virus.

According to a further aspect, the invention relates to the use of at least one antigen conferring protection against tetanus, at least one antigen conferring protection against diphtheria and at least one antigen conferring protection against tick-borne encephalitis for the preparation of the combination vaccine for the prophylactic treatment of tetanus, diphtheria and tick-borne encephalitis.

The expression "Lf" as used herein is the abbreviation of the so-called Limes flocculationis. 1 Lf is defined as the amount of a toxin (or a toxoid) which leads to flocculation in the presence of 1 IU antitoxin. Thus, the Lf units indicate the content of specific protein in a sample. An amount of an antigen, such as a toxin (or a toxoid) quantity, is usually expressed as Lf or Lf/ml. The determination of the Lf value is based on the observation of Ramon (1922) according to which floccution occurs when toxin (or toxoid) and antitoxin are mixed in equivalent amounts. This means, Lf refers to the amount of antigen which precipitates in the presence of a defined amount of antibody. The Lf can be determined by methods common in the field of vaccine preparation, for example by Rocket electrophoresis (see Michael Schwanig,,Diphtherie und Tetanus: Wie häufig braucht der Mensch Boosterdosen?" in: Alte und neue Impfstoffe in Deutschland. Grundlagen für kunftige Entscheidungen, 27-33 (2001), Infomed Medizinische Verlagsgesellschaft mbH, Berlin.

As described above, the term Lf defines the amount of toxoid which is measured by physicochemical methods. However, the development of vaccines with antigens adsorbed to alumimum hydroxide having a strongly enhanced effectiveness over vaccines with antigens not adsorbed to aluminium hydroxide made it necessary to provide a new evaluation method for vaccines, since the binding value determined by the flocculation method does not represent a reliable value for the immunologic response. Thus, for assessment of the efficacy of a (toxoid) vaccine, the potency is much more relevant than the amount of Lf in a given vaccine (see Michael Schwanig "Diphtherie und Tetanus: Wie häufig braucht der Mensch Boosterdosen?" in: Alte und neue Impfstoffe in Deutschland. Grundlagen für künftige Entscheidungen, 27-33 (2001), Infomed Medizinische Verlagsgesellschaft mbH, Berlin. The potency of a vaccine is referred to in International Units (usually per ml (IU/ml) or per dose (IU/dose)). As used herein the "potency of a vaccine" is defined as the effectiveness of a given antigen or vaccine relative to a standard vaccine calibrated in IU. Determination of the potency is conducted according to well-known methods, for example in animal models or cell cultures. Further details can be inferred from the European Pharmacopoeia, 5^{th} edition 5.0) .

The term "combination vaccine", as used herein, refers to a vaccine which confers protection against more than one infectious disease. The term embraces, for example, vaccines comprising antigens conferring protection against more than one disease caused by a pathogenic microorganism. Thus, the combination vaccine may confer protection against two, three, four, five or six different diseases. Accordingly, such vaccines are referred to as bivalent, trivalent, tetravalent, pentavalent, and hexavalent, respectively. In contrast, a vaccine which confers protection against only one specific disease is referred to as monovalent herein. Regularly, combination vaccines comprise several antigens which originate from different organisms.

According to the invention, a combination vaccine is provided which contains at least one antigen which is suitable to induce an immunological response in a subject (such as a mammal) upon administration, wherein this response is such that the subject is protected against tetanus; at least one further antigen which is suitable to induce an immunological response in that subject upon co-administration, wherein this response is such that the subject is protected against diphtheria; and at least one antigen which is suitable to induce an immunological response in that subject upon administration, wherein this response is such that the subject is protected against tick-borne encephalitis. Preferably, the antigens achieve their protective effect in the immunized subject by inducing specific antibodies against these antigens (active immunity).

It has been found that the combination vaccines according to the present invention may be combined with one or more further antigens and remain effective and stable. The term "stable" as used in the context of the present invention means that the combination vaccine formulation can be kept for a period of eight days or more preferably 14 days at room temperature without any substantial loss with respect to immunogenity and stability of its distinct antigen compounds. In order to enhance stability, stabilizing agents such as saccharose can be added to the vaccine composition. Instead of saccharose or in addition thereto, other stabilisers, e.g. human serum albumin, mannose, trehalose, mannite, or polygeline can also be added as stabilizing agents. The vaccines of the present invention exhibit a pH value of preferably between 5.0 to 8.0, more preferably between 6.0 to 7.0, wherein a pH value of 6.8 to 7.8 is most preferred.

The term "effective" as used herein, refers to the fact that the antigen upon single or repeated administration to a subject (for example, a mammal) confers protection against a specific disease. As used herein the term "conferring protection" means that the antigen, upon administeration, induces an immunological response in the vaccinated subject which response is capable to protect said subject from the symptoms of subsequent infection. As used herein "immunologocal response" means that the immune system of the subject adapts to the antigen so that upon contact of the subject with the pathogenic microorganism, parts or products of that organism the symptoms associated with the disease that is caused by that organism are reduced or completely eliminated. Preferably, the immunological response induced in the vaccinated subject is based on the production of specific antibodies which are directed against the pathogenic microorganism, parts thereof, or against metabolic products produced by that microorganism, such as toxins. Preferably, the tetanus and/or diphtheria antigens induce the production of antibodies to an extend which gives rise to an geometric antibody titer with respect to that antigen of at least 0.01 IU/ml to 2.0 IU/ml, preferably 0.1 IU/ml or more and most preferably 1.0 IU/ml or more for at least 14 days after vaccination. Briefly, minimal protection is reached when the geometric antibody titer is about 0.01 IU/ml, whereas long-term protection is achieved when the titers are about 0.5 IU/ml, preferably about 1.0 IU/ml. The determination of the antibody titer of the vaccinated person can be performed by use of common methods. Preferably, the degree of protection which is reached upon administration is as high as that achieved by administering mono-, bi- or trivalent vaccines, for example a DT-combination, a TBE vaccine, or a Td-TBE combination vaccine

In the context of the present invention, the term "antigen" refers to any substance derived from a disease-causing microorganism which is upon administration suitable to confer protection against that specific disease. Particularly, the term refers to a substance originating from a pathogenic microorganism, preferably from bacteria, protozoa, viruses or fungi, which upon administration (for example, by transdermal or intramuscular injection, or oral uptake) is capable of inducing the formation of antibodies against this microorganism, parts of said microorganism or metabolic products of this organism, in a subject such as a mammal; this type of immunity is referred to as active immunity herein.

The antigen can be akilled, attenuated or inactivated viruses or a killed, attenuated or inactivated bacteria or a protein, polypeptide, peptide, glycoprotein, or a fragment thereof, a lipid, oligosaccharide, polysaccharide, lipopolysaccharide. It will be appreciated that also fragments, derivatives and other modified forms of such molecules may be used as antigens. Moreover, the antigen can be a nucleic acid, such as a deoxynucleic acid or a ribonucleic acid. Vaccines based on DNA or RNA have attracted much interest in the recent years. In this approach, the nucleic acid comprised by the vaccine is translated to the respective protein or polypeptide in the subject's body after administration. In the case of DNA or RNA vaccines, it is particularly advantageous that antigenicity of the nucleic acid can be modified by simple sequence modifications.

The antigens may be obtained in pure form from different manufacturers, or may be prepared by purifying the respective antigenic molecules from a suitable source. If the antigen is, for example, a bacterial exopolypeptide which is secreted by the producing organism, it may be readily purified from culture supernatants by common chromatographic methods (for example, the diphtheria toxin). Alternatively, if the antigen is a nucleic acid, it may be obtained by methods involving polymerase-chain-reaction etc. If the antigen is a whole virus fraction, it may be purified, for example, from a cell culture supernatant after infection and lysis of the cells by use of seperators and filters. Further examples of antigens and means for their isolation and purification methods have been described by von Behring et al., Sera und Impfstoffe. In: Ullmanns Encyklopadie der technischen Chemie. Band 21, 273-310 (1982), Verlag Chemie GmbH, Weinheim. Alternatively, peptide, polypeptide or protein antigens can also be produced by recombinant DNA-techniques, such as heterologous expression. Methods suitable for such production processes are known in the art.

It will be appreciated by those skilled in the art that antigenic molecules such as peptides, polypeptides or proteins may also be produced by recombinant DNA-techniques, such as heterologous expression. Methods suitable for such production processes are well known in the art and comprise, for example, the baculovirus expression system, the yeast-two-hybrid system, chinese-hamster-ovary (CHO) system and many others.

The term "antigen" in particular embraces exoproteins or exopolypeptides which are secreted by microorganisms, such as bacteria. Also embraced are endoproteins or endopolypeptides which are released from the producing cell upon lysis. Such exoproteins/exopolypeptides or endoproteins/endopolypeptides can be, for example, protein toxins or peptide toxins. In a vaccine, these toxins are preferably used in a modified form, because in many cases the pure toxin is not suitable for direct injection. Here, the toxins are usually modified chemically so that they retain their antigenicity but loose (part of) their toxicity. Such modified toxins are usually referred to as toxoids. The formation of toxoids may be achieved, for example, by treating a toxin with formaldehyd which blocks some of the free amino groups in the molecule, thereby converting the toxin to a toxoid. Preferably, the toxoid is much less effective compared to the corresponding toxin and therefore can be given safely and in high doses. Examples of toxoid antigens are the diphtheria toxoid and the tetanus toxoid. Methods of producing toxoids are well known in the art and are summarized, for example, in described by von Behring et al., see above. It will be appreciated that also fragments of the polypeptide toxins or toxoids, in unmodified or modified form, may be used in practising the present invention.

Where immunization against whole microorganisms is aimed at, the antigen according to the invention can be a part of the microorganism, such as a specific protein of the outer membrane of gramnegative bacteria, or the microorganism itself. In the latter case, the microorganism can be killed by agents such as formaldehyd, phenol or heat, and the dead cells can be subsequently incorporated as an antigen into a vaccine and injected. In these cases, the antigen consists of a whole cell/virus fraction or of an extract of such a fraction. This mode of vaccination is, for example, regularly applied with pathogenic organisms such as Vibrio cholerae, Bordetella pertussis and Yersinia pestis.

According to a preferred embodiment of the invention, the at least one antigen conferring protection against tetanus in the combination vaccine comprises a tetanus toxoid. According to a further preferred embodiment of the invention, the at least one antigen conferring protection against diphtheria in the combination vaccine comprises a diphtheria toxoid. This means, that the combination vaccine according to the present invention may, next to other antigenic substances, may comprise toxoids which can be obtained from toxin-producing strains of Clostridium tetani and Corynebacterium diphtheriae.

Generally, toxoids can be prepared from toxins in cultures of Clostridium tetani and Corynebacterium diphtheriae. The toxins produced by these organisms have been described with respect to their primary structure; see for example Eisel, U et al., EMBO J. 1986 Oct; 5(10):2495-502.

The toxoids of Corynebacterium diphtheriae and Clostridium tetani can also be purchased in the form of a DT combination vaccine (DT-Impfstoff Behring für Kinder; Diphtherie-Tetanus-Adsorbat-Impfstoff für Kinder (Chiron Behring GmbH & Co KG) which contains 20 Lf tetanus toxoid with a potency of at least 40 IU/dose and 20 Lf diphtheria toxoid with a potency of at least 30 IU/dose. Additionaly, the combination vaccine Td-pur® (Chiron Behring GmbH & Co KG) contains 20 Lf tetanus toxoid with a potency of at least 20 IU/dose and only 1.5 Lf diphtheria toxoid having a potency of at least 2 IU/dose. According to the invention, these vaccines or the antigens which are components of these vaccines, can be directly used as starting materials for preparing the combination vaccines of the invention. Alternatively, as mentioned above, the toxoids can also be prepared by purification of the toxins from the supernatant of cultures of the respective organisms. Subsequently, the purified toxins can be modified according to methods known in the art in order to produce toxoids.

According to the invention, the at least one antigen conferring protection against tetanus (for example, the tetanus toxoid) may be present in an amount which corresponds to a potency of about 1-70 IU/dose. When used in a combination vaccine for children under 12 years of age, the antigen may be present in an amount which corresponds to a potency of about 20-60 IU/dose, whereas a potency of about 30-50 IU/dose is preferred, and a potency of at least 40 IU/dose is most preferred. When used in a combination vaccine for children of at least 12 years of age and adults the antigen may be present in an amount which corresponds to a potency of about 5-40 IU/dose, whereas a potency of about 10-30 IU/dose is preferred, and a potency of at least 20 IU/dose is most preferred.

Similarly, the at least one antigen conferring protection against diptheria (for example, the diphtheria toxoid) may be present in an amount which corresponds to a potency of 0.1-70 IU/dose. When used in a combination vaccine for children under 12 years of age, the antigen may be present in an amount which corresponds to a potency of about 10-50 IU/dose, whereas a potency of about 20-40 IU/dose is preferred, and a potency of at least 30 IU/dose is most preferred. When used in a combination vaccine for children of at least 12 years of age and adults the antigen may be present in an amount which corresponds to a potency of about 0.5-10 IU/dose, whereas a potency of about 0.75-5 IU/dose is preferred, and a potency of at least 2 IU/dose is most preferred.

According to a preferred embodiment of the invention, the at least one antigen in the combination vaccine conferring protection against tetanus is derived from Clostridium tetani strain Massachusetts F1. The strain can be obtained from Commonwealth of Massachusetts, Departure of Publich Health, Division Biologic Laboratories, Boston. Furthermore, the at least one antigen conferring protection against diphtheria is preferably derived from Corynebacterium diphtheriae strain Massachusetts 8 Park Williams (see J.H. Mueller, 1939, J. Immunol. 37, 103-111; Russell, L., and R. Holmes, 1985, Infect. Immun. 47:575-578). This strain can be obtained from Massachusetts Antitoxin and Vaccine Laboratory, Forest Hills, Boston.

In the case of virus vaccines, the antigen of the vaccine can consist of a whole virus fraction or an extract of a whole virus fraction as well as of distinct pure molecules derived from the virus such as DNA, RNA, proteins, peptides or fragments of proteins and peptides. As used herein, the expression "whole virus fraction" means that the virus, which is isolated for the vaccine formulation is not treated such that a subunit antigen fraction is prepared. In contrast, the complete fraction contains the virus obtained from a culture after separating from the medium components. The complete fraction can alsobe used as an antigen. Also embraced by the term are fractions which are enriched with respect to a certain antigen. For example, in the case of the TBE-virus, a fraction is used which is enriched with respect to glycoprotin E; however the fraction also contains other virus components, i.e. it is not a fraction containing purified virus. Processes and means for obtaining a "whole virus fraction" are described, for example, in "Vaccines", Plotkin and Orenstein (eds.), 2004.

According to a preferred embodiment of the invention, the at least one antigen conferring protection against tick-borne encephalitis is characterized in that it originates from TBE-flavivirus strain Neudörfl or TBE-flavivirus strain K23, both of which are known with respect to their sequence in the prior art (Heinz et al., J. Med. Virol. 1980; 6: 213-221; Klockmann et al., J. Biol. Standard. 1989; 17: 331-342). However, also other strains of the TBE-virus can be used for obtaining an antigen against TBE, for example, strains Louping Ill, Petracova, Sofyn.

According to a preferred embodiment of the invention, the at least one antigen conferring protection against tick-borne encephalitis derives from a TBE-flavivirus, i.e. it is an antigen of a TBE-virus. According to a particularly preferred embodiment of the invention, the antigen from a TBE-flavivirus is the envelope glycoprotein E or fragments thereof. The primary structure of this glycoprotein is known in the prior art and it is described for example in the publication of Ecker et al., J. Gen. Virol. 80, 179-185 (1999). According to an alternative embodiment of the invention, the antigen of the TBE-virus is a whole virus fraction.

According to the present invention, the amount of said at least one antigen conferring protection against tick-borne encephalitis, such as the antigen derived from a TBE-virus, may range in the combination vaccine from about 0.01 µg/dose to about 10 µg/dose, if a whole virus vaccine such as Encepur® is used. Alternatively, in case pure components of the virus are used, the amount depends on the nature of the antigen molecule. If, for example, purified glycoprotein E should be incorporated into the combination vaccine, an amount of about 0.1 to 5 µg/dose, preferentially 0.5 to 2.5 µg/dose may be used. Preferably, the antigen conferring protection against tick-borne encephalitis is present in an amount of 1.5 µg/dose.

According to a preferred aspect, the invention provides a combination vaccine, wherein the at least one antigen conferring protection against tetanus is present in an amount which corresponds to a potency of at least 20 IU/dose, the at least one antigen conferring protection against diphtheria is present in an amount which corresponds to a potency of at least 2 IU/dose, and the at least one antigen conferring protection against tick-borne encephalitis is present in an amount of at least 0.75 µg/dose.

As used herein, the term "dose" refers the amount of a medicament or vaccine which is to be administered. Specifically, the term refers to a dosage unit form of a medicament or vaccine. For example, if the medicament in question is a liquid, the dose will be referred to in terms of the volume. Suitable doses in the context of the present invention have a volume of 0.1-2.0 ml, preferably 0.5-1.0 ml. If the medicament in question is present as a pill, the dose will be referred to in terms of the number of pills which is to be administered. In the context of the present invention, the term dose is preferably used to address a volume which is to be administered. As used herein, the potency of the combination vaccine according to the invention is referred to as IU/dose. This means that the potency as expressed by the specific IU value applies if the dose is administered. For example, if a vaccine exhibits a potency of 20 IU/dose and the dose is present in a volume of 1 ml, an effect of protection is achieved which corresponds to 20 IU (relative to the respective calibration vaccine) when the complete 1 ml is administered.

The combination vaccine according to the invention can further comprise at least one additional antigen conferring protection against a further disease, for example, an antigen from another pathogenic microorganism, such as a virus and/or a bacterial pathogen, so that tetravalent, pentavalent or hexavalent vaccines can be produced. Preferably, the one or more additional antigen is capable of conferring protection against a disease or medical condition selected from a group of pertussis, polio, hepatitis A, meningococcal diseases, Lyme disease. Examples for such multivalent vaccines which can be prepared in accordance with the present invention comprise (but are not restricted to) a Td-aP-TBE vaccine, a Td-IPV-TBE vaccine, and a Td-aP-IPV-TBE vaccine.

Generally the optimum amount of a specific antigen can be evaluated using standard methods involving measuring the antibody titers as well as other responses of the vaccinated subject. The vaccination course can be adopted to the proposal of national or international authorities. It is expected that the antigens of the combination vaccine according to the invention are used in an amount which produces immunological responses which confer protection. Specifically, these amounts produce geometric mean titers of antibodies against the specific antigen (tetanus or diphtheria) of approximately at least 0.01 IU/ml preferably at least 0.1 IU/ml and most preferably at least 1.0 IU/ml for at least 14 days after vaccination.

According to a preferred embodiment of the present invention, the antigens of Clostridium tetani, Corynebacterium diphtheriae, and the TBE-virus are present in the vaccine absorbed to an adjuvant. Adjuvants are frequently used in the formulation of vaccine compositions. In the context of the present invention, the term "adjuvant" refers to any substance which leads to an enhanced immunological reaction or response of the subject which receives the immunization as compared to administering the vaccine without the adjuvant. Such an enhanced response can be observed, for example, as an enhanced antibody titer against the respective antigen.

Adjuvants for use with the invention include, but are not limited to, one or more of the following: mineral containing compositions, such as aluminum salts and/or calcium salts; oil-emulsions, such as squalene-water emulsions, for example MF59 (5% Squalene, 0.5% Tween 80, and 0.5% Span 85; Saponin formulations including QS7, QS17, QS18, QS21, QH-A, QH-B and QH-C; immunostimulatory oligonucleotides, such as sequences containing a CpG motif; bioadhesives and mucoadhesives, such as esterified hyaluronic acid microspheres (Singh et *al.* (2001) J. *Cont. Rele.* 70:267-276) or mucoadhesives such as cross-linked derivatives of poly(acrylic acid), polyvinyl alcohol, polyvinyl pyrollidone, polysaccharides and carboxymethylcellulose; biodegradable microparticles such as particles of a poly-α-hydroxy acid, a polyhydroxybutyric acid, a polyorthoester, a polyanhydride, a polycaprolactone, etc.; liposomes; polyoxyethylene ethers and polyoxyethylene esters (see for example WO99/52549), polyphosphazene (PCPP) formulations; muramyl peptides; imidazoquinolone compounds; human immunomodulators such as interleukins *(e.g.* IL-1, IL-2, IL-4, IL-5, IL-6, IL-7, IL-12, *etc.),* interferons (e.g. interferon-γ), macrophage colony stimulating factor, and tumor necrosis factor; and others.

Mineral containing compositions which include mineral salts, such as aluminum salts and/or calcium salts are particularly preferred in the present invention as adjuvants. The invention includes mineral salts such as hydroxides (e.g. oxyhydroxides), phosphates (e.g. hydroxyphosphates, orthophosphates), sulfates, etc. (e.g. see chapters 8 & 9 of *Vaccine Design* (1995) eds. Powell & Newman. ISBN: 030644867X. Plenum.), or mixtures of different mineral compounds (e.g. a mixture of a phosphate and a hydroxide adjuvant, optionally with an excess of the phosphate), with the compounds taking any suitable form (e.g. gel, crystalline, amorphous, etc.), and with adsorption to the salt(s) being preferred. The mineral containing compositions may also be formulated as a particle of metal salt (see, for example, WO 00/23105). According to a preferred embodiment of the present invention, the adjuvant used is an aluminium salt. According to a particular preferred embodiment of the present invention, the aluminium salt used is aluminium hydroxide. The precise amount of adjuvant to be used depend on its nature. For example, if aluminium hydroxide is the adjuvant, it can be used in an amount of 2-20 g/l antigen suspension.

The combination vaccines of present invention can be prepared readily according to different methods, all of which are known in the art. Processes and means for the production vaccines are described, for example, in "Vaccines", Plotkin and Orenstein (eds.), 2004. Further, the "Impfcodex, Impfung für Kinder, Erwachsende und Reisende", 5^{th} edition, Chiron Behring GmbH & Co (ed.), 2001 provides useful information. The components of the vaccine can, for example, be mixed before adsorbing the mixture to an appropriate adjuvant. However, as will be appreciated by the those skilled in art, each antigen component can also be adsorbed separately to an adjuvant prior to mixing this component with the other antigen components.

If, for example, the combination vaccine Td-IPV-TBE should be formulated, the individually concentrated IPV antigens of serotype 1, 2, and 3 can be added either to the premixed Td-TBE combination or to the premixed Td combination. In the latter case, the TBE concentrate is added at last. Alternatively, the three individually IPV antigens are mixed to a three-valent IPV concentrate, which is then added to the Td- or Td-TBE premixture. The manufacturing process is similar, if pertussis antigens compounds are to be added in order to create a Td-aP-TBE or a Td-aP-IPV-TBE vaccine. The pertussis antigen containing vaccine may comprise a whole cell fraction, an acellular product or a recombinantly produced product. Preferably, the pertussis component is the pertussis toxoid or a fragment thereof, the filamenteous haemaglutinin antigen, or a protein of the outer membrane.

According to a further embodiment, the different antigen formulations are blended before bottling the combination vaccine. This means, the different antigen formulations can be mixed to a combined multivalent vaccine, and subsequently absorbed to a suitable carrier/adjuvant, such as aluminum hydroxide.

The production process has to be performed in accordance with the guidelines of good laboratory practice. For example, the mixing of different antigen formulations has to be performed under sterile conditions so that the resulting vaccine is not contaminated with hazardous substances such as infective agents which would be harmful to the immunized subject. Generally, mixing and bottling of the combination vaccine can be performed according to methods well known in the art.

According to a further aspect, the invention relates to a kit comprising at least one antigen conferring protection against tetanus, at least one antigen conferring protection against diphtheria; and at least one antigen conferring protection against tick-borne encephalitis and in addition further reagents which are suitable for preparing a combination vaccine according to the invention. Preferably, the kits of the invention comprise at least one antigen of Clostridium tetani, at least one antigen of Corynebacterium diphtheriae, and at least one antigen of a TBE-virus. Additional reagents can comprise, for example, buffers, stabilizing agents, anti-oxidants and/or preservatives.

Preferably, the antigens of the combination vaccine are stored separately for instantaneous mixing before injection. Here, the antigens can be present in separate vials, either as a solution or in lyophilized form for reconstitution. According to a further embodiment, the antigen formulations can be present in a syringe with two or more separate chambers. Upon injection, these chambers are joined so that the different antigen formulations mix up shortly before injection to the combination vaccine of the present invention.

According to a further aspect, the invention relates to a method for preparing a combination according to the invention, in which at least one antigen conferring protection against tetanus, at least one antigen conferring protection against diphtheria, and at least one antigen conferring protection against tick-borne encephalitis are mixed and the mixed antigens are subsequently absorbed to a suitable adjuvant. Alternatively, the invention relates to a method for preparing a combination according to the invention, in which at least one antigen conferring protection against tetanus, at least one antigen conferring protection against diphtheria, and at least one antigen conferring protection against tick-borne encephalitis are absorbed separately from each other to a suitable adjuvant and the adsorbed antigens are subsequently mixed with each other.

The invention will be further illustrated by the following non-limiting following examples.

### Examples

### Example 1: Production of a Td-TBE combination vaccine

This example discloses manufacturing of a Td-TBE combination vaccine. Preferentially, all the antigens are individually adsorbed to aluminium hydroxide for at least 30 minutes.

Tetanus and diphtheria toxoids are obtained and purified according to methods well known in the art. Briefly, common semi-synthetic media are inoculated with Clostridium tetani (Harvard strain) after 1 to 2 passages as a pre-culture into a fermenter. The cells are cultured for 6 to 7 days. Tetanus toxin is produced within the first three days after inoculation. The toxin is released in the medium after lysis of the cells. At the end of the culturing phase, the toxin is obtained by sterile filtration and detoxified by the addition of formalin and incubation at 37° C for 3-4 weeks. Toxoid concentrate is obtained by ultrafiltration and salt precipitation. Similarly, cultures of Corynebacterium diphtheriae (strain Park & Williams, BW 8) is inoculated into a fermenter after 1-2 pre-culture passages. The cells are cultured for 48 hours under stirring and strong aeration. The culturing temperature usually is 33°-37°C. Diphtheria toxin is secreted by cell into the culture supernatant. The toxin is obtained by sterile filtration and is usually incubated (without the addition of formalin) for several weeks. Subsequently, formalin is added and the toxin is detoxified for six weeks by incubation at 37°C.

Purification and concentration is performed by ultrafiltration and ammoniumsulphate precipitation.

Alternatively, one can also use ready-to-use combination vaccines such as Td-pur® (Chiron Behring GmbH & Co KG) or DT Impfstoff für Kinder (Chiron Behring GmbH & Co KG) as a starting material. Briefly, Td-pur® comprises about 20 IU/dose tetanus toxoid (which corresponds to about 20 Lf) and about 2 IU/dose diphtheria toxoid (which corresponds to about 1.5 Lf). In comparison, the DT Impfstoff für Kinder contains at least 40 IU/dose tetanus toxoid (which corresponds to about 20 Lf) and at least 30 IU/dose diphtheria toxoid (which corresponds to about 20 Lf ) . In each case, a concentration of 15 g/l aluminium hydroxide is used.

In order to prepare the vaccine, tetanus antigen is adsorbed to the aluminium hydroxide to give a concentrate of 750 Lf/ml. Similarly, the diphtheria toxoid is adsorbed to give a concentrate of 300 Lf/ml. Finally, purified TBE-whole virus fraction is used to give a concentrate of 60 pg/ml. The tetanus and diphtheria toxoid suspensions are 75 fold concentrated compared to the concentration of the final combination vaccine, and the TBE antigen concentration is 20 fold.

The concentration of aluminium hydroxide is 15 g/l for each concentrate. These concentrates are transferred to a formulation vessel (Hermann Waldner GmbH & Co KG, Wangen, Germany or Pharmatec Deutschland GmbH, Dresden, Germany) and then an appropriate amount of aluminium hydroxide suspension (15 g/l) is added to give a final concentration of 2.0 mg aluminium hydroxide/ml in the final combination vaccine. Then, sterile filtrated saccharose solution is added to give a final concentration of 50 mg/ml and sterile filtrated NaCl solution and water for injection is added to fill the master batch to the final volume. If necessary, the pH value is adjusted to 6.8-7.8. and the master batch is agitated for 30 minutes. Then, the TBE-Td vaccine portions were bottled into vials or syringes. Several further combination vaccines can be produced according to this process, inter alia, Td-TBE-aP, Td-TBE-IPV and Td-TBE-aP-IPV combinations.

### Example 2: Modes of administration and storing

Different modes of storing/administration may be used in view of putative differences with respect to the effectiveness of the combination vaccine. Briefly, the following approaches may be used:
- A.: Td and TBE antigens are combined in a two-chamber vaccine syringe (Becton-Dickinson) or two vials, such that the two liquid components are stored separately in the device, and the antigens are instantaneously at the time of injection.
- B.: One of the two antigen components (either the Td or the TBE component) is lyophilised by use of a lyophilizer (Hof-Sonderanlagenbau, Lohra, Germany), while the other antigen is obtained in liquid form from the process described in example 1. The lyophilised component is reconstituted by the liquid component prior to the immunization.
- C.: The Td and the TBE antigens are blended in one suspension in a syringe or a vial and the mixture is stored for 7 days at 4°C prior to the immunization.

### Example 3: Production of a tetravalent or pentavalent Td-TBE combination vaccines

As described in Example 1, all components are seperately adsorbed to aluminium hydroxide and subsequently mixed.

Instead of using Td for blending with the TBE component according to the above mentioned examples, a trivalent component like Td-aP (Td together with acellular pertussis antigen; obtainable, for example, as monovalent vaccine Acelluvax of Chiron Vaccines, Chiron S.p.A., Siena, Italy or Td-IPV (inactivated polio virus; antigen obtainable, for example, from The Netherland Vaccine Institute, Bilthoven, The Netherland) may be used resulting in a tetravalent Td-TBE-aPor Td-TBE-IPV vaccine, respectively. In addition, an even broader combination is possible, e.g. Td-TBE-aP-IPV vaccine.

## Claims

1. Combination vaccine comprising
a) at least one antigen conferring protection against tetanus,
b) at least one antigen conferring protection against diphtheria; and
c) at least one antigen conferring protection against tick-borne encephalitis.

2. Combination vaccine according to claim 1, wherein said at least one antigen conferring protection against tetanus is an antigen of Clostridium tetani, said at least one antigen conferring protection against diphtheria is an antigen of Corynebacterium diphtheriae, and said at least one antigen conferring protection against tick-borne encephalitis is an antigen from a tick-borne encephalitis-virus.

3. Combination vaccine according to claim 1 or 2, wherein said at least one antigen conferring protection against tick-borne encephalitis is from TBE-virus strain Neudörfl or TBE-virus strain K23.

4. Combination vaccine according to claims 1 to 3, wherein said at least one antigen conferring protection against tick-borne encephalitis is the envelope glycoprotein E of a TBE-virus.

5. Combination vaccine according to claims 1 or 3, wherein said at least one antigen conferring protection against tick-borne encephalitis is a whole virus fraction of a TBE-virus.

6. Combination vaccine according to claim 4, wherein said at least one antigen conferring protection against tick-borne encephalitis is present in an amount of about 0.1 to 5 µg/dose, preferably 0.5 to 2.5 µg/dose.

7. Combination vaccine according to claim 5, wherein said at least one antigen conferring protection against tick-borne encephalitis is present in an amount of 0.01 µg/dose to about 10 µg/dose.

8. Combination vaccine according to claims 1 to 7, wherein said at least one antigen conferring protection against tetanus is derived from Clostridium tetani strain Harvard.

9. Combination vaccine according to claims 1 to 8, wherein said at least one antigen conferring protection against tetanus comprises a tetanus toxoid.

10. Combination vaccine according to claim 1 to 9, wherein said at least one antigen conferring protection against tetanus is present in an amount which corresponds to a potency of about 10-60 IU/dose, preferably 10-50 IU/dose and more preferably at least 20 IU/dose.

11. Combination vaccine according to claims 1 to 10, wherein said at least one antigen conferring protection against diphtheria is derived from Corynebacterium diphtheriae strain Massachusetts 8 Park Williams.

12. Combination vaccine according to claims 1 to 11, wherein said at least one antigen conferring protection against diphtheria comprises a diphtheria toxoid.

13. Combination vaccine according to claim 1 to 12, wherein said at least one antigen conferring protection against diphtheria is present in an amount which corresponds to a potency of about 10-50 IU/dose, preferably 20-40 IU/dose and more preferably at least 30 IU/dose.

14. Combination vaccine according to claim 1 to 12, wherein said at least one antigen conferring protection against diphtheria is present in an amount which corresponds to a potency of about 0.5-10 IU/dose, preferably 0.75-5 IU/dose and more preferably at least 2 IU/dose.

15. Combination vaccine according to any of claims 1 to 12, wherein said at least one antigen conferring protection against tetanus is present in an amount which corresponds to a potency of at least 20 IU/dose, said at least one antigen conferring protection against diphtheria is present in an amount which corresponds to a potency of at least 2 IU/dose, and said at least one antigen conferring protection against tick-borne encephalitis is present in an amount of 0.75 µg/dose.

16. Combination vaccine according to one of the preceding claims, wherein the vaccine further comprises an adjuvant.

17. Combination vaccine according to claim 16, wherein said adjuvant is an aluminium salt, such as aluminiumhydroxide.

18. Combination vaccine according to one of the preceding claims, wherein the vaccine further comprises at least one additional antigen from another pathogenic microorganism.

19. Combination vaccine according to claim 18, wherein said at least one additional antigen is capable of conferring protection against a disease or medical condition selected from a group of pertussis, polio, hepatitis A, meningococcal diseases or Lyme disease.

20. Combination vaccine according to claim 19, wherein said at least one additional antigen is capable of conferring protection against pertussis.

21. Use of at least one antigen conferring protection against tetanus, at least one antigen conferring protection against diphtheria and at least one antigen conferring protection against tick-borne encephalitis for the preparation of a combination vaccine for the prophylactic protection of tetanus, diphtheria and tick-borne encephalitis.

22. Use according to claim 21, wherein said antigens are antigens of Clostridium tetani, Corynebacterium diphtheriae and the TBE-virus.

23. Kit comprising at least one antigen conferring protection against tetanus, at least one antigen conferring protection against diphtheria and at least one antigen conferring protection against tick-borne encephalitis and reagents suitable for preparing a combination vaccine.

24. Kit according to claim 23, wherein said antigens are antigens of Clostridium tetani, Corynebacterium diphtheriae and the TBE-virus.

25. Kit according to claims 23 or 24, wherein said antigens are separately stored.

26. Kit according to claim 25, wherein the antigens are present in a syringe with two or more separate chambers.

27. Kit according to claim 25, wherein the antigens are present in two or more separate vials.

28. Method for preparing a combination vaccine of one of claims 1 to 20, comprising the steps of:
a) mixing at least one antigen conferring protection against tetanus, at least one antigen conferring protection against diphtheria, and at least one antigen conferring protection against tick-borne encephalitis; and
b) adsorbing the mixed antigens to a suitable adjuvant.

29. Method for preparing a combination vaccine of one of claims 1 to 20, comprising the steps of:
a) adsorbing at least one antigen conferring protection against tetanus, at least one antigen conferring protection against diphtheria, and at least one antigen conferring protection against tickborne encephalitis separately from each other to a suitable adjuvant; and
b) mixing the adsorbed antigens with each other.

30. Method according to claims 28 or 29, wherein said at least one antigen conferring protection against tetanus is an antigen of Clostridium tetani, said at least one antigen conferring protection against diphtheria is an antigen of Corynebacterium diphtheriae, and said at least one antigen conferring protection against tick-borne encephalitis is an antigen from a TBE-virus.
